(19) Europäisches Patentamt
European Patent Office
Office européen des brevets

(11) Numéro de publication : **0 378 455 B1**

# (12) FASCICULE DE BREVET EUROPEEN

(45) Date de publication du fascicule du brevet : **27.04.94 Bulletin 94/17**

(51) Int. Cl.$^5$ : **C07D 281/10,** // C07C323/36

(21) Numéro de dépôt : **90400028.8**

(22) Date de dépôt : **05.01.90**

(54) **Procédé de préparation de dérivés de (+)-(2S,3S)-3-hydroxy-2-(4-méthoxyphényl)2,3-dihydro-5H-1,5-benzothiazépine-4-one.**

(30) Priorité : **11.01.89 FR 8900246**

(43) Date de publication de la demande : **18.07.90 Bulletin 90/29**

(45) Mention de la délivrance du brevet : **27.04.94 Bulletin 94/17**

(84) Etats contractants désignés : **AT BE CH DE DK ES FR GB GR IT LI LU NL SE**

(56) Documents cités : **JOURNAL OF THE CHEMICAL SOCIETY, PERKIN TRANSACTIONS 1, no. 3, mars 1985, pages 421-427, Londres, GB; T. HASHIYAMA et al.: "Reaction of 3-phenylglycidic esters. Part 2. Stereo- and regio-selectivity in the oxirane ring opening of methyl trans-3-(4-methoxyphenyl)glycidate with various thiophenols and the effects of solvent and temperature"**

(73) Titulaire : **SYNTHELABO**
**22, Avenue Galilée**
**F-92350 Le Plessis Robinson (FR)**

(72) Inventeur : **Rossey, Guy**
**10, rue Paul Verlaine**
**F-78960 Voisins Le Bretonneux (FR)**
Inventeur : **Chekroun, Isaac**
**25bis, rue des Econdaux**
**F-93800 Epinay (FR)**
Inventeur : **Ugolini, Antonio**
**8, rue de Seine**
**F-78230 Le Pecq (FR)**
Inventeur : **Wick, Alexander**
**10, Boulevard des Plants**
**F-78860 St Nom La Breteche (FR)**
Inventeur : **Gerin, Bernard**
**14, rue des Crosnières, Résidence Mansart**
**F-78200 Mantes La Jolie (FR)**
Inventeur : **Bourbon, André**
**13, Avenue Paul Bert**
**F-78200 Mantes-La-Jolie (FR)**
Inventeur : **Graux, Jean-Baptiste**
**Chemin de Puyet, Cidex 11, Denguin**
**F-64230 Lescar (FR)**

(74) Mandataire : **Ludwig, Jacques et al**
**SYNTHELABO, Service Brevets, B.P. 72**
**F-92352 Le Plessis Robinson Cédex (FR)**

EP 0 378 455 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet un procédé de préparation de dérivés de (+)-(2S,3S)-3-hydroxy-2-(4-méthoxyphényl)-2,3-dihydro-5*H*-1,5-benzothiazépine-4-one éventuellement chlorés sur le noyau aromatique.

Ces composés, optiquement purs, sont des intermédiaires de synthèse de composés à activités thérapeutiques tels que la (+)-(2S,3S)-3-acétyloxy-5-(2-diméthylaminoéthyl)-2-(4-méthoxyphényl)-2,3-dihydro-5*H*-1,5-benzothiazépine-4-one.

Le schéma de réaction du procédé est donné à la page suivante. Dans les formules, X représente un atome d'hydrogène ou d'halogène.

La première étape consiste à faire réagir un 2-aminothiophénol de formule générale (II) avec le (-)-(2R,3S)-2,3-époxy-3-(4-méthoxyphényl)propionate de méthyle de formule (III). Par ouverture du cycle époxyde on obtient le (2S,3S)-3-[(2-aminophényl)thio]-2-hydroxy-3-(4-méthoxyphényl)propionate de méthyle de formule générale (IV).

La deuxième étape consiste à cycliser ce composé en présence d'acide.

Les principes de réaction de chacune des deux étapes sont bien connus.

On les trouve par exemple dans Chem. Pharm. Bull., 18, 2028-2037 (1970), où l'ester de formule (III) est utilisé sous forme racémique. La première étape nécessite plusieurs heures de chauffage à 150-160°C et, après séparation et purification de l'ester de formule générale (IV), on effectue la seconde étape en hydrolysant cet ester et en cyclisant l'acide obtenu en présence d'acide sulfurique ou acétique, dans le xylène au reflux.

Le brevet des Etats-Unis d'Amérique 4416819 décrit la première étape du procédé, où l'ester de formule (III) (racémique) réagit avec l'aminothiophénol (II) dans le toluène après 6h de chauffage au reflux.

Schéma

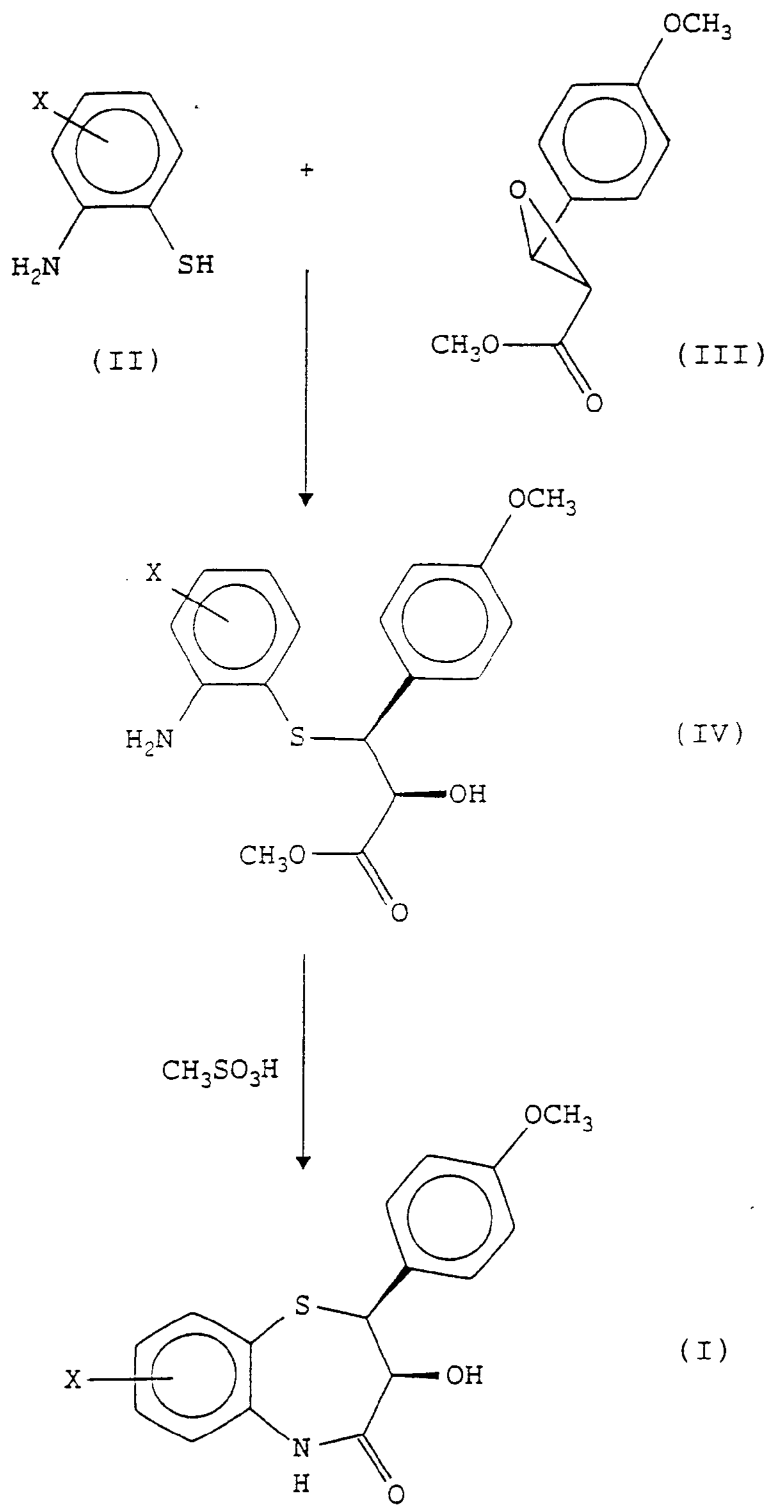


(II) (III) (IV) (I)

La demande de brevet japonais 145160/1986, qui décrit la synthèse de l'ester de formule (III) optiquement

pur, décrit également la réaction de ce dernier avec un aminothiophénol de formule générale (II), en 10h de chauffage au reflux dans le toluène.
Enfin la demande de brevet européen 0154838 décrit, entre autres, un procédé réunissant les deux étapes. Les réactions s'effectuent sans solvant, nécessitent 16h de chauffage à 160°C, et fournissent un mélange d'isomères optiques de composé final et de composé intermédiaire.

Ainsi il est clair que tous les procédés connus sont inadaptés à une fabrication industrielle rentable de composés de formule générale (I), en raison des divers inconvénients qui les accompagnent : rendements médiocres, températures élevées, nécessité de purifier les composés intermédiaires et/ou finals, temps de réactions longs.

C'est pourquoi la présente invention propose un procédé remédiant aux inconvénients de la technique connue, en apportant les avantages suivants :

- les deux étapes de réaction peuvent être effectuées dans un seul et même réacteur, sans qu'il soit donc nécessaire de vidanger et nettoyer ce dernier entre les deux étapes, ou d'utiliser un second réacteur (pour des raisons d'ergonomie on peu préférer réaliser chaque étape dans un réacteur différent, mais en tous cas il n'est pas nécessaire d'isoler le composé intermédiaire, l'opération consistant alors en un simple transvasement de l'ensemble du mélange réactionnel) ;
- le rendement global est élevé par rapport à ceux des procédés connus ;
- la consommation d'énergie est réduite, en particulier au cours de la première étape ;
- les temps de réaction sont courts ;
- le composé final est pur.

Les conditions physico-chimiques du procédé selon l'invention, qui permettent de réunir les avantages énumérés ci-dessus, sont décrites ci-après.

L'ester de départ, de formule (III), est utilisé sous forme optiquement pure. Il est décrit dans les demandes de brevets japonais 145159/1986, 145160/1986 et 145174/1986.
La possibilité d'effectuer les deux étapes de la réaction dans un seul récipient, ou de ne pas isoler le composé intermédiaire, est due au choix d'un solvant unique, qui convient bien à chacune des deux étapes.
Des solvants spécifiques à chacune des deux étapes sont certes déjà connus (dichloroéthane, toluène, xylène, etc) mais, étant différents pour chaque étape, ils ne permettent pas le déroulement de l'ensemble du procédé dans le même réacteur et, en tous cas, impliquent la nécessité d'isoler le composé intermédiaire.

Les solvants à utiliser selon l'invention sont les solvants chlorés ayant un point d'ébullition supérieur à 70°C. Des exemples de tels solvants sont le 1,2,3-trichloropropane, les dichlorobenzènes et, de préférence, le chlorobenzène.

Ces solvants sont non seulement - et de manière inattendue très favorables à un bon rendement global mais, en outre, ils sont tellement efficaces que la première étape du procédé ne nécessite qu'un chauffage de mise en route, le caractère exothermique de la réaction étant suffisant pour son entretien sans apport d'énergie extérieure. Cette particularité était tout à fait imprévisible, car elle n'a jamais été constatée dans le cas d'autres solvants.
De plus, l'emploi de ces solvants favorise la sélectivité thréo/érythro de la première étape. Dans le cas d'autres solvants, on constate en effet qu'on obtient un mélange de diastéréoisomères de formule générale (IV).

Une autre particularité de l'invention tient au catalyseur utilisé dans la seconde étape du procédé. Alors qu'il est connu que la cyclisation s'effectue mieux en milieu acide (sulfurique ou acétique), le catalyseur à utiliser selon l'invention est l'acide méthanesulfonique, l'acide phosphorique, l'acide trifluoroacétique, l'acide trifluorométhanesulfonique, l'acide perchlorique ou l'acide paratoluènesulfonique. Le catalyseur préféré est l'acide méthanesulfonique. Ces acides, qui permettent d'obtenir un excellent rendement, sont simplement à ajouter au milieu réactionnel dès que la première étape est achevée.

Les exemples qui vont suivre illustrent en détail des modes de mise en oeuvre du procédé selon l'invention.

Exemple 1

Dans un réacteur émaillé de 25 l, purgé à l'azote, on introduit 3 kg de (-)-(2R,3S)-2,3-époxy-3-(4-méthoxyphényl)propionate de méthyle et 10 l de chlorobenzène, et on chauffe à 100°C. On arrête le chauffage et, en l'espace de 30mn, pour ne pas laisser la température dépasser 120°C, on introduit une solution de 1907 g de 2-aminothiophénol dans 1,5 l de chlorobenzène, puis encore 3,5 l de chlorobenzène pour rincer l'ampoule d'introduction et les canalisations.
On maintient la température à environ 115°C pendant encore 30mn, puis on ajoute 37,5 ml d'acide méthanesulfonique et on chauffe le mélange au reflux pendant 8h, en éliminant par distillation un mélange de méthanol et de chlorobenzène, pour ne pas laisser la température descendre en dessous de 132°C (point d'ébullition du chlorobenzène).

On arrête le chauffage, on laisse le mélange revenir à 20°C, on le refroidit à 5°C pendant 1h, on filtre les cristaux formés, en les rinçant avec du chlorobenzène, et on les sèche sous vide à 100°C.
On obtient ainsi 3463 g de (+)-(2S,3S)-3-hydroxy-2-(4-méthoxyphényl)-2,3-dihydro-5*H*-1,5-benzothiazépine-4-one pure.
Point de fusion : 200,3-201,8°C. $[\alpha]_D^{20}$=+114° (c=0,1 ; DMF).

Exemple 2

Dans un ballon de 1 l placé sous argon on introduit 50 g de (-)-(2R,3S)-2,3-époxy-3-(4-méthoxyphényl)propionate de méthyle et 350 ml de dichlorobenzène (produit du commerce constitué d'isomères ortho, méta et para), et on chauffe le mélange à 115°C. Puis, en l'espace de 30mn, pour ne pas laisser la température dépasser 120°C, on introduit 31,8 g de 2-aminothiophénol.
On maintient la température à 120°C pendant encore 30mn, puis on ajoute 0,62 ml d'acide méthanesulfonique et on chauffe le mélange à 150°C pendant 3h (la réaction est en fait achevée au bout de 2h).
On laisse le mélange revenir à température ambiante, on le refroidit à 5°C pendant 1h, on filtre les cristaux formés en les lavant au dichlorobenzène, et on les sèche sous vide.
On obtient 49,5 g de (+)-(2S,3S)-3-hydroxy-2-(4-méthoxyphényl)-2,3-dihydro-5*H*-1,5-benzothiazépine-4-one pure.
Point de fusion : 203-204°C.

Exemple 3

On ajoute 18,8 g de 2-aminothiophénol goutte à goutte à une solution de 30 g de (-)-(2R,3S)-2,3-époxy-3-(4-méthoxyphényl)propionate de méthyle dans 200 ml de 1,2,3-trichloropropane chauffée à 110-115°C.
Après 30mn on ajoute 0,35 g d'acide méthanesulfonique, et on chauffe le mélange à 145-150°C pendant 6h.
On laisse le mélange revenir à température ambiante, on le refroidit, on le filtre en lavant les cristaux avec du 1,2,3-trichloropropane, et on les sèche sous vide à 50°C.
On obtient 33,8 g de (+)-(2S,3S)-3-hydroxy-2-(4-méthoxyphényl)-2,3-dihydro-5*H*-1,5-benzothiazépine-4-one pure.
Point de fusion : 202,8-204,1°C.

Exemple 4

En opérant de la manière décrite dans l'exemple 1, et en utilisant du 2-amino-5-chlorothiophénol et du (-)-(2R,3S)-2,3-époxy-3-(4-méthoxyphényl)propionate de méthyle, on obtient la (+)-(2S,3S)-8-chloro-3-hydroxy-2-(4-méthoxyphényl)-2,3-dihydro-5*H*-1,5-benzothiazépine-4-one.
Point de fusion : 237-241°C. $[\alpha]_D^{20}$ = +91,9° (c=0,1 ; DMF).

## Revendications

1. Procédé de préparation de composés de formule générale (I)

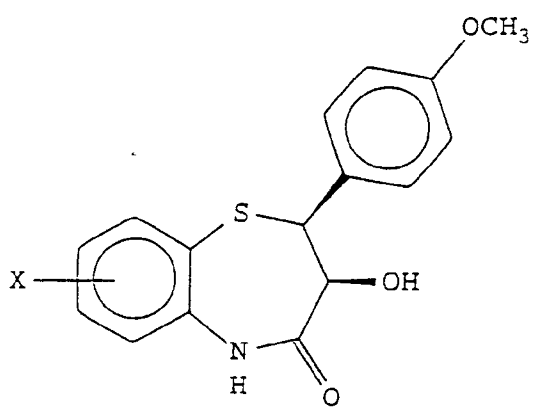

(I)

dans laquelle X représente un atome d'hydrogène ou de chlore, consistant à faire d'abord réagir un 2-aminothiophénol de formule générale (II)

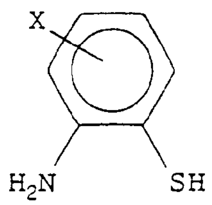

(II)

dans laquelle X est tel que défini ci-dessus,
avec le (-)-(2R,3S)-2,3-époxy-3-(4-méthoxyphényl)propionate de méthyle de formule (III)

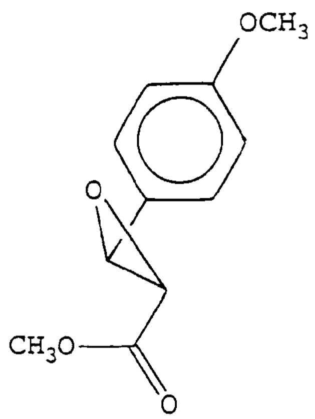

(III)

puis à cycliser le composé intermédiaire ainsi obtenu, de formule générale (IV)

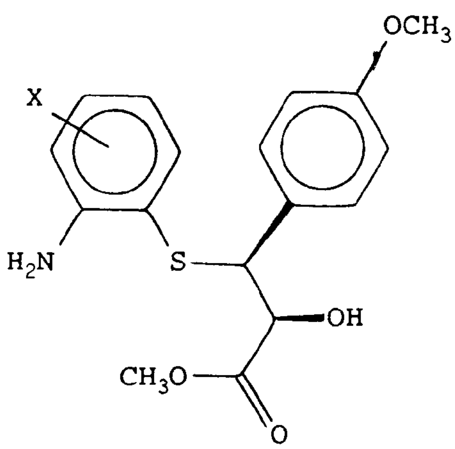

(IV)

en présence d'acide, procédé caractérisé en ce qu'on effectue les deux étapes de réaction sans isoler le composé intermédiaire, en utilisant un solvant choisi parmi le chlorobenzène, les dichlorobenzènes et le 1,2,3-trichloropropane.

2. Procédé selon la revendication 1, caractérisé en ce que X représente un atome d'hydrogène.

3. Procédé selon la revendication 1, caractérisé en ce que X représente un atome de chlore.

4. Procédé selon la revendication 1, caractérisé en ce que le solvant est le chlorobenzène.

5. Procédé selon la revendication 1, caractérisé en ce que le solvant est un dichlorobenzène.

6. Procédé selon la revendication 1, caractérisé en ce que le solvant est le 1,2,3-trichloropropane.

7. Procédé selon la revendication 1, caractérisé en ce qu'on utilise, pour catalyser la cyclisation du composé intermédiaire de formule générale (IV), l'acide méthanesulfonique, l'acide phosphorique, l'acide trifluoroacétique, l'acide trifluorométhanesulfonique, l'acide perchlorique ou l'acide paratoluènesulfonique.

**Patentansprüche**

1. Verfahren zur Herstellung von Verbindungen der allgemeinen Formel (I)

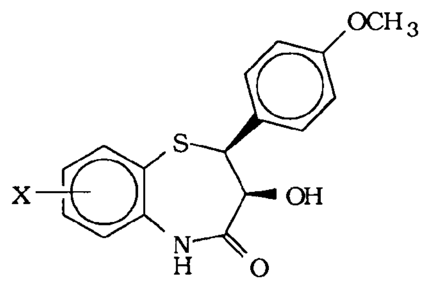

(I)

in der X ein Wasserstoffatom oder ein Chloratom bedeutet, welches darin besteht, zunächst ein 2-Aminothiophenol der allgemeinen Formel (II)

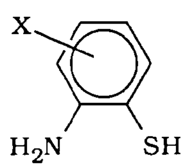

(II)

in der X die oben angegebenen Bedeutungen besitzt,
mit (-)-(2R,3S)-2,3-Epoxy-3-(4-methoxyphenyl)-propionsäuremethylester der Formel (III)

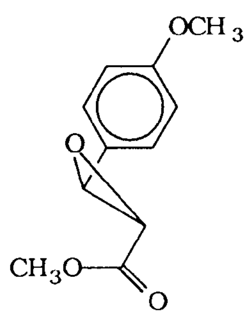

(III)

umzusetzen und dann die in dieser Weise erhaltene Zwischenverbindung der allgemeinen Formel (IV)

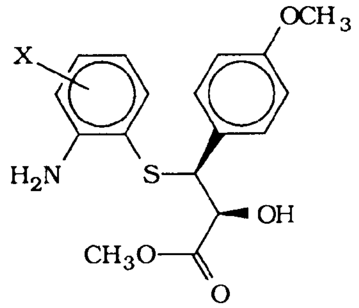

(IV)

in Gegenwart einer Säure zu cyclisieren, **dadurch gekennzeichnet,** daß man die beiden Stufen der Reaktion ohne Isolierng der Zwischenverbindung durchführt unter Verwendung eines Lösungsmittels ausgewählt aus Chlorbenzol, Dichlorbenzolen und 1,2,3-Trichlorpropan.

2. Verfahren nach Anspruch 1, **dadurch gekennzeichnet,** daß X ein Wasserstoffatom bedeutet.

3. Verfahren nach Anspruch 1, **dadurch gekennzeichnet,** daß X ein Chloratm bedeutet.

4. Verfahren nach Anspruch 1, **dadurch gekennzeichnet,** daß das Lösungsmittel Chlorbenzol ist.

5. Verfahren nach Anspruch 1. **dadurch gekennzeichnet**, daß das Lösungsmittel Dichlorbenzol ist.

6. Verfahren nach Anspruch 1, **dadurch gekennzeichnet,** daß das Lösungsmittel 1,2,3-Trichlorpropan ist.

7. Verfahren nach Anspruch 1, **dadurch gekennzeichnet,** daß man zur Katalyse der Cyclisierung der Zwischenverbindung der allgemeinen Formel (IV) Methansulfonsäure. Phosphorsäure, Trifluoressigsäure. Trifluormethansulfonsäure, Perchlorsäure oder p-Toluolsulfonsäure verwendet.

## Claims

1. A process for the preparation of compounds of general formula (I)

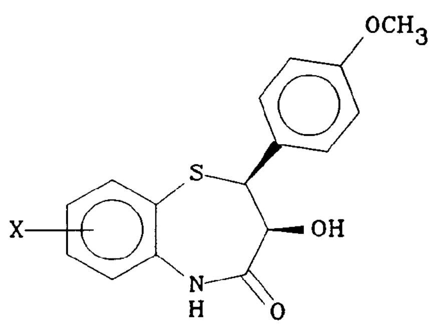

(I)

wherein X represents a hydrogen or chlorine atom,
comprising first reacting a 2-aminothiophenol of general formula (II)

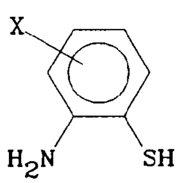

(II)

wherein X is as defined above,
with methyl (-)-(2R,3S)-2,3-epoxy-3-(4-methoxyphenyl)propionate of formula (III)

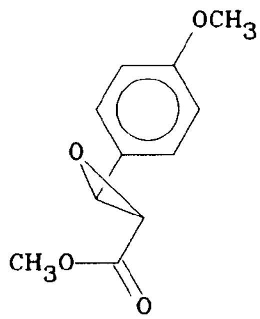

(III)

and then cyclizing the intermediate compound, of general formula (IV)

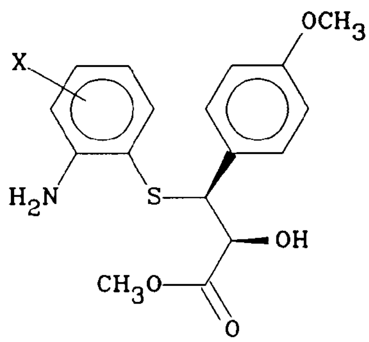

(IV)

thus obtained in the presence of an acid, the process being characterized in that the two reaction steps are performed without isolation of the intermediate compound, by using a solvent selected from chlorobenzene, dichlorobenzenes and 1,2,3-trichloropropane.

**2.** A process according to claim 1, characterized in that X represents a hydrogen atom.

**3.** A process according to claim 1, characterized in that X represents a chlorine atom.

**4.** A process according to claim 1, characterized in that the solvent is chlorobenzene.

**5.** A process according to claim 1, characterized in that the solvent is a dichlorobenzene.

**6.** A process according to claim 1, characterized in that the solvent is 1,2,3-trichloropropane.

**7.** A process according to claim 1, characterized in that methanesulphonic acid, phosphoric acid, trifluoroacetic acid, trifluoromethanesulphonic acid, perchloric acid or paratoluenesulfonic acid is used to catalyse the cyclization of the intermediate compound of general formula (IV).